(19) Europäisches Patentamt
European Patent Office
Office européen des brevets

(11) Publication number: 0 008 167
A1

# (12) EUROPEAN PATENT APPLICATION

(21) Application number: 79301383.0

(22) Date of filing: 12.07.79

(51) Int. Cl.³: C 07 D 311/92

(30) Priority: 15.08.78 GB 3343078
14.03.79 GB 7908909

(43) Date of publication of application: 20.02.80
Bulletin 80/4

(84) Designated Contracting States: AT BE CH DE FR GB IT LU NL SE

(71) Applicant: FISONS LIMITED, Fison House 9 Grosvenor Street, London (GB)

(72) Inventor: Allan, Douglas Neil, 16 Buttermere Court, Congleton, Cheshire (GB)
Inventor: Barton, John, 29 Gables Lea Sutton Bonington, Loughborough Leicestershire (GB)
Inventor: Bell, John David, 64 Brook View Drive, Keyworth, Nottingham (GB)
Inventor: Fanthorpe, Michael Morris, 9 Petworth Drive, Thorpe Acre, Loughborough Leicestershire (GB)
Inventor: Townend, John, 16 Brookfield Drive, Holmes Chapel, Crewe Cheshire (GB)

(74) Representative: Craig, Christopher Bradberry et al, Fisons Limited Fison House Princes Street, Ipswich 1P1 1QH (GB)

## (54) Improved process for the production of 6,7,8,9-tetrahydro-5-hydroxy-4-oxo-10-propyl-4H-naphtho(2,3-b)pyran-2-carboxylic acid.

(57) There is described an improved process for the production of the biologically active compound 6,7,8,9-tetrahydro-5-hydroxy-4-oxo-10-propyl-4H-naphtho [2,3-b] pyran-2-carboxylic acid of the formula

CH₂–CH₂–CH₃
O
COOH
OH
O

which comprises hydrolysis of 6,7,8,9-tetrahydro-5-diazo-4-oxo-10-propyl-4H-naphtho [2,3-b] pyran-2-carboxylic acid or an ester thereof, characterised in that the hydrolysis is carried out in the presence of less than 10% w/w nitrite anion. The diazo compound may be made by diazotisation of 6,7,8,9-tetrahydro-5-amino-4-oxo-10-propyl-4H-naphtho [2,3-b] pyran-carboxylic acid or an ester thereof in the presence of nitrous acid.

EP 0 008 167 A1

BA 33430/78

This invention relates to an improved process.

6,7,8,9-Tetrahydro-5-hydroxy-4-oxo-10-propyl-4H-naphtho[2,3-b]-pyran-2-carboxylic acid is a known compound having biologically useful properties, which has previously been made by diazotisation of 6,7,8,9-tetrahydro-5-amino-4-oxo-10-propyl-4H-naphtho[2,3-b]-pyran-2-carboxylic acid ethyl ester using a nitrite salt and sulphuric acid, followed by hydrolysis of the resulting diazonium intermediate. However this known process produces an impure product containing resinous (and highly coloured) by products, 6,7,8,9-tetrahydro-4-oxo-10-propyl-4H-naphtho[2,3-b]pyran-2-carboxylic acid, and ethyl 6,7,8,9-tetrahydro-5-hydroxy-4-oxo-10-propyl-4H-naphtho[2,3-b]pyran-2-carboxylate.

The known process also produces the product in the form of very fine particles which are difficult to separate and purify (occluded impurities) and which when dry form very fine dusts, thus causing considerable handling problems. The removal of the undesirable impurities from the product of the known process by crystallisation has not proved practically possible and complex and expensive processes involving esterification and re-hydrolysis have proved necessary to reduce these impurities to an acceptable level.

We have now found a process which is adapted to produce material of greater purity and in a more easily handleable form.

According to the invention we provide a process for the production of 6,7,8,9-tetrahydro-5-hydroxy-4-oxo-10-propyl-4H-naphtho[2,3-b]-

pyran-2-carboxylic acid, which comprises hydrolysis of 6,7,8,9-tetrahydro-5-diazo-4-oxo-10-propyl-4H-naphtho[2,3-b]pyran-2-carboxylic acid or an ester thereof, characterised in that the hydrolysis is carried out in the presence of less than 10% w/w of nitrite anion.

We prefer to carry out the hydrolysis in the presence of less than 5% w/w, more preferably less than 10 ppm and most preferably the substantial absence of nitrite anion.

The nitrite ion content may be measured using conventional techniques. The quantity of nitrite anion specified in this description and in the claims is measured as the total available nitrite whether organic or inorganic, ionised or unionised and expressed as if it were sodium nitrite. The w/w % and ppm are expressed as nitrite/solution. The substantial absence of nitrite anion may be detected by a negative response to starch/iodide paper.

The diazo compound may be made by diazotisation of 6,7,8,9-tetrahydro-5-amino-4-oxo-10-propyl-4H-naphtho[2,3-b]pyran-2-carboxylic acid, or preferably an ester, e.g. a lower alkyl ester such as the ethyl ester thereof, in the presence of nitrous acid, which may be produced by admixture of a nitrite salt and an acid. The process of producing the diazo compound usually involves the use of excess nitrite and under normal circumstances the diazo compound is produced in admixture with excess nitrite. We prefer to remove this excess nitrite soon after the diazo compound has been formed, to avoid continued contact between the diazo compound and the nitrite.

Excess nitrite may be removed from the diazo compound in a variety of ways, e.g. the addition or presence of a compound which will react with the nitrite to form a compound which can easily be removed from the reaction mixture, or which is inert under the reaction conditions. Examples of such compounds include urea; sulphamic acid or an alcohol, for example a C 1 to 6 alkanol, e.g. butanol, propanol or preferably methanol or ethanol.

The effectiveness of lower alkanols in reducing the levels of impurities is particularly surprising as aqueous alkanols are conventionally thought to induce breakdown of diazo compounds to the corresponding -H substituted compound, not, as in the present case, to enhance the production of the -OH substituted compound.

When using urea we prefer to use an excess of urea, e.g. from 0.01 to 0.25 moles of urea for each mole of the diazonium compound. When using an alcohol we prefer to use at least sufficient to react with the nitrite anion, e.g. up to 2 moles and preferably from 0.1 to 1 mole of alcohol per mole of the diazonium compound. When using sulphamic acid we prefer to use just sufficient sulphamic acid to react with the excess nitrite.

We prefer to use a lower alkanol to remove the excess nitrite, and to use urea during the hydrolysis step as we have found that their use can lead to a purer product and also that the presence of urea during the hydrolysis step can induce crystallisation of the product acid during hydrolysis thus leading to a more easily separated and a less easily contaminated product.

According to a further facet of the invention we therefore provide a process for the production of 6,7,8,9-tetrahydro-5-hydroxy-4-oxo-10-propyl-4H-naphtho/_2,3-b_7pyran-2-carboxylic acid, which comprises hydrolysis of 6,7,8,9-tetrahydro-5-diazo-4-oxo-10-propyl-4H-naphtho/_2,3-b_7pyran-2-carboxylic acid or an ester thereof, characterised in that the hydrolysis is carried out in such a manner that the product acid crystallises during the hydrolysis.

We prefer to effect the lowering of the level of the excess nitrite as a separate stage; however the lowering may take place in the diazotisation medium and/or vessel, or in the hydrolysis medium and/or vessel, or in an entirely separate vessel.

Once the nitrite level has been lowered the diazo compound may be stored for reasonable periods without substantial deterioration.

In making the diazo compound we prefer to use an ester of 6,7,8,9-tetrahydro-5-amino-4-oxo-10-propyl-4H-naphtho/_2,3-b_7pyran-2-carboxylic acid and for the ester to be a lower alkyl ester, e.g. the ethyl ester.

In the diazotisation step the nitrite salt may conveniently be sodium nitrite and is preferably used in excess, e.g. up to 15% excess, to ensure completeness of production of the diazo compound.

The acid used in the production of the diazo compound may conveniently be sulphuric acid. The diazotisation process is preferably carried out in an aqueous acidic medium at a temperature of less than 5°C.

When an alkanol is used the removal of the (gaseous) reaction

product of the alkanol and the nitrite may be enhanced by use of an inert gas purge, e.g. a nitrogen purge, or by the application of a vacuum.

The hydrolysis of the diazo compound preferably takes place in an aqueous medium and preferably in the presence of the same acid as is used in the preparation of the diazo compound. In particular we prefer to use the sulphuric acid of concentration between 25% and 75%, and more preferably about 50%.

The hydrolysis preferably takes place at an elevated temperature, e.g. a temperature of from 100 to 200°C, preferably from 115 to 140°C. For each initial part by weight of diazo compound in the hydrolysis step we prefer to use from 10 to 50 parts by weight of 70% sulphuric acid, and from 0.5 to 5.0 parts by weight of urea.

When an ester starting material is used, as is preferred, the hydrolysis comprises a simultaneous hydrolysis of the diazo group and of the ester group.

Thus in practice the ester of 6,7,8,9-tetrahydro-5-amino-4-oxo-10-propyl-4H-naphtho[2,3-b]pyran-2-carboxylic acid may be dissolved in the acid and a concentrated solution of the nitrite salt in water added to the acid solution with cooling to maintain the reaction at a temperature of below about 5°C. The reaction mixture is maintained

below 5°C until substantially all the ester of 6,7,8,9-tetrahydro-5-amino-4-oxo-10-propyl-4H-naphtho/‾2,3-b‾/-pyran-2-carboxylic acid has been reacted. The alcohol, urea or sulphamic acid is then added (the last two preferably in solution) and the reaction mixture stirred, or otherwise agitated, for sufficient time to allow decomposition or removal of the nitrite. The nitrite reduced reaction product is then added to preheated 70% sulphuric acid (optionally containing urea) and maintained as an elevated temperature, e.g. of 115-140°C to effect hydrolysis. When the hydrolysis of the diazonium intermediate is complete the reaction mixture may be mixed with water and the solid product may be separated, washed with water, and, if desired or necessary be recrystallised optionally with charcoal treatment.

It is to be understood that urea can serve either to reduce the nitrite content, or to assist crystallisation of the product or both.

The nitrite level in the diazonium compound may also be reduced by passing the diazo compound in a thin film over a heated surface. This process will normally involve simultaneous hydrolysis of the diazo compound.

The temperature of the surface is preferably from about 100 to 200°C, more preferably from 140 to 160°C and, depending on the temperature, the residence time of the reaction mixture on the surface may vary from about 10 seconds to 5 minutes, preferably 10 seconds to 2 minutes. The hydrolysis may if desired be carried out under an inert atmosphere, e.g. of nitrogen. The reaction mixture flowing

off the surface may be mixed with water and the solid product may be separated, washed with water, and, if desired or necessary be recrystallised, e.g. from methyl ethyl ketone, optionally with charcoal treatment.

The reactor for present use is one wherein a film of the reaction mixture moves over a heated surface. It is usually desirable that the film, when formed, be driven across the heated surface. This may be achieved by scraper bars moving across a flat surface or by the action of an archimedean screw within a heated tubular reactor. A particularly preferred reactor is a glass tube having an internal diameter of about 2 inches (5 mm) and a length of about 8 feet (2.4 metres), which is inclined at an angle of about $9^{o}$ to the horizontal and is adapted to be rotated, e.g. at from about 50 to 150 r.p.m. It will be appreciated that the dimensions of the reactor, the clearance between the scrapers and the heated surface, the inclination and speed of rotation of the reactor may be varied to suit the reaction mixture fed thereto.

The method by which the heating of the surface is achieved is conventional, for example by electrical or other means. In the case of a rotating tubular glass reactor it is preferred to use infra-red heating.

The invention is illustrated, by in no way limited by the following Examples, in which 'sulphuric acid' means 98% sulphuric acid.

Example 1

(Heated surface)

The ethyl ester of 6,7,8,9-tetrahydro-5-amino-4-oxo-10-propyl-4H-naphtho[2,3-b]pyran-2-carboxylic acid (50 gm: 0.152 mole) was dissolved in a mixture of sulphuric acid (0.424L) and water (0.182L). To this was added a solution of sodium nitrite (13.25 gm: 0.167 mole) in water (21 ml) such that the temperature remained below $0^{o}C$. After ½ hour stirring at $0^{o}C$, thin layer chromatography indicated none of the starting ester present. The solution was then diluted with a mixture of sulphuric acid (0.560L) and water (0.190L) and run down the inside of a rotating glass tube under the following conditions:-

| | | |
|---|---|---|
| Angle of inclination of the tube | = | $8.9^{o}$ |
| Temperature | = | $145 \pm 2^{o}C$ |
| Input/min | = | $200 \pm 10$ drops |
| Output | = | $10/90 \pm 10$ secs |
| Average residence time | = | 30 - 40 secs |

The solution from the apparatus after breakdown was run into water (dilution x5). The solid obtained as a yellow precipitate was filtered off, slurried with demineralised water (3L), washed with demineralised water to pH 3 and dried in vacuo at $70^{o}C$.

Yield = 39.8 gm (86.7% theoretical)

10g of the above product was recrystallised from methyl ethyl ketone (150 ml) and water (10 ml) with a 5% charcoal screen to give 8.5 gm material, which was recrystallised again from methyl ethyl

ketone (130 ml) and water (8.5 ml) with a 20% charcoal screen to give 5.1 gm of solid 6,7,8,9-tetrahydro-5-hydroxy-4-oxo-10-propyl-4H-naphtho-[2,3-b]pyran-2-carboxylic acid (60% return).

Example 2

(Urea)

Ethyl 6,7,8,9-tetrahydro-5-amino-4-oxo-10-propyl-4H-naphtho-[2,3-b]pyran-2-carboxylate (11.4g) (0.035 mole) was dissolved in 50% aqueous sulphuric acid (114 ml) and cooled to 0°C. A solution of sodium nitrite (2.78g, 0.04 mole) in water (5 ml) was added drop-wise with stirring and cooling over 30 minutes so that the temperature did not exceed 5°C. The mixture was then stirred for a further 15 minutes at 0°C. The mixture was then slowly added, with stirring, to a solution of urea (20g) in 50% aqueous sulphuric acid (200 ml) heated to 110°-120°C, ensuring that a temperature of at least 110° was maintained. The mixture was then heated for a further 30 minutes at 100°-120°C. The mixture was cooled to 50°C and added to water (500 ml), with stirring and cooling. The precipitated solid was filtered off, washed with water and dried. 6,7,8,9-Tetrahydro-5-hydroxy-4-oxo-10-propyl-4H-naphtho[2,3-b]pyran-2-carboxylic acid was obtained as an amber solid, mpt 264-266°C (8.6g).

Example 3

(Ethanol and urea)

To a pale orange solution of ethyl 6,7,8,9-tetrahydro-5-amino-4-oxo-10-propyl-4H-naphtho[2,3-b]pyran-2-carboxylate (84g,

0.24 moles) in aqueous sulphuric acid (916 ml sulphuric acid added to water to give 1200 ml at room temperature) and ethanol (26 ml) at 4°C was added a solution of sodium nitrite (25.3g, 0.37 moles) in water (42 ml) over 45 minutes keeping the temperature at 4 to 6°C. The cloudy orange solution was stirred cold for a further 1½ hours, then was added to aqueous sulphuric acid (1184 ml of sulphuric acid added to water to give 1800 ml at room temperature) containing urea (168g) at 135 to 125° over 30 minutes. Some orange solid formed during the addition and a deep orange mixture was obtained. This was allowed to cool to 60°C, then was poured with stirring into water (5 litres) to give a yellow precipitate. After cooling to room temperature and standing overnight the yellow solid was filtered off and washed with water (5 litres). The solid was oven dried to a deep yellow powder 77.8g.

Example 4

(Ethanol and urea)

To a pale orange solution of ethyl 6,7,8,9-tetrahydro-5-amino-4-oxo-10-propyl-4H-[2,3-b]pyran-2-carboxylate (21.0g, 63.8 m moles) in aqueous sulphuric acid (228 ml sulphuric acid added to water to give 300 ml at 20°) at 3°C, was added an aqueous solution of sodium nitrite (5.1g, 73.9 m moles) in water (10.5 ml) over 30 minutes at 3-5°C. The mixture was stirred for 1 hour and ethanol (4.0 ml, 68.5 m moles) was added. Evolution of a gas commenced and stirring was continued for 30 minutes during which time the temperature was allowed to rise to 18°C. Nitrogen gas was passed through the yellow

mixture for 15 minutes. The solution was then added to a stirred aqueous sulphuric acid (sulphuric acid (297 ml) and water (195 ml)) containing urea (45g) at 122-125°C. The resulting orange suspension was allowed to cool to 60°C and poured into water (1275 ml) and the mixture cooled to 30°C. The solid was filtered off, washed with water (1275 ml) and dried, 17.33g (90%).

Example 5

(Ethanol and urea)

To a pale orange solution of 6,7,8,9-tetrahydro-5-amino-4-oxo-10-propyl-4H-naphtho/_2,3-b_7pyran-2-carboxylate (21.0g, 63.8m moles) in denatured ethanol (4.0 ml, 68.5m moles) and cold aqueous sulphuric acid (228 ml sulphuric acid added to water, 95 ml, to give 300 ml at 20°C) cooled to 3°C, was added an aqueous solution of sodium nitrite (5.1g, 73.9m moles in 10.5 ml water) over 40 minutes keeping the temperature at 3 to 5°C. The mixture was sampled after 5 minutes, the sample being diluted and spotted on starch iodide paper. This indicated an excess of nitrite. The sample, was shown by thin layer chromatography to contain no starting amine. After stirring for 1 hour, a gas being evolved and the temperature being allowed to rise to 10°C a similar sample showed only a trace of nitrite. The mixture was then flushed with nitrogen for 2 hours the temperature being allowed to rise to 16°C, giving an orange cloudy solution. Sampling indicated absence of nitrite. The solution was added over 50 minutes to aqueous sulphuric acid (297 ml sulphuric acid added to water, 195 ml, to give 450 ml at 20°C) containing urea (63g) at 130° to

128°C. Orange solid started crystallising early in the addition and at the end of addition an orange solution containing a considerable amount of orange solid was obtained. This was allowed to cool to 60°C over 1 hour, (more solid forming), then was poured into water (1275 ml) with rapid stirring to give a deep yellow precipitate. The mixture was allowed to cool with stirring to 30°C, then was filtered and the solid washed with water (1275 ml) and oven dried overnight to a yellow/orange powder 18.26g (94%).

Example 6

(Sulphamic acid titrated)

To a pale orange solution of 6,7,8,9-tetrahydro-5-amino-4-oxo-10-propyl-4H-naphtho[2,3-b]pyran-2-carboxylate (21.0g, 63.8m moles) in cold aqueous sulphuric acid (228 ml of sulphuric acid added to water, 85 ml, and cooled to room temperature) cooled to ~3°C was added an aqueous solution of sodium nitrite (5.1g, 73.9m moles in 10.5 ml of water) over 30 minutes keeping the temperature at 3 to 5°C. The mixture was stirred at ~5°C for 30 minutes, then was treated dropwise over 30 minutes at 5 to 8°C with aqueous sulphamic acid (5.2ml of 0.93g, 9.6m moles in 10 ml of water; i.e. 5.0m moles) until a diluted ~~sample~~ showed absence of nitrous acid. The cloudy orange/yellow mixture was allowed to warm to 19°C with a nitrogen flush over 2¼ hours, and was then added over 30 minutes to aqueous sulphuric acid (297 ml of sulphuric acid added to water, 195 ml to give 450 ml at room temperature) containing urea (45g) at 127 to 131°C. The resulting mixture (dark orange solution and

solid) was allowed to cool to 60°C over 1 hour, and was then poured into water (1275 ml) with rapid stirring to give a mustard precipitate. The mixture was allowed to cool with stirring, then after standing overnight was filtered (fairly fast filtering), the solid washed with water (1275 ml) and oven dried to a mustard powder, 17.90g (3%).

Example 7

(Urea)

To a pale orange solution of 6,7,8,9-tetrahydro-5-amino-4-oxo-10-propyl-4H-naphtho[2,3-b]pyran-2-carboxylate (21.0g, 63.8m moles) in cold aqueous sulphuric acid (228 ml of sulphuric acid added to water, 90 ml, and cooled to room temperature) cooled to ~3°C was added an aqueous solution of sodium nitrite (5.1g, 73.9m moles, in 10.5 ml of water) over 30 minutes keeping the temperature at 3 to 5°C. The mixture was stirred at ~5°C for 45 minutes and was then treated over 30 minutes with aqueous urea (0.60g, 10m moles in 10 ml of water) the temperature rising to 11°C. As excess nitrous acid was still present after warming to 19°C over 2 hours more aqueous urea (3.2g, 53m moles in 5 ml of water) was added. The temperature rose to 23°C, and fell back to 19°C over 1 hour, the excess nitrous acid slowly reducing to an undectable level over this time.

The cloudy orange/yellow mixture was added over 30 minutes to aqueous sulphuric acid (297ml of sulphuric acid added to water 195 ml to give 450 ml at room temperature) containing urea (45g) at 125 to 128°C. The resulting mixture (orange solution and solid) was allowed

to cool to 60$^{o}$C over 1 hour then was poured into water (1275 ml) with rapid stirring to give a yellow precipitate. The mixture was allowed to cool with stirring then, after standing overnight, was filtered (fairly fast filtering), the solid washed with water (1275m moles) and oven dried to give a yellow powder 18.05g (94%).

What we claim is:-

1. A process for the production of 6,7,8,9-tetrahydro-5-hydroxy-4-oxo-10-propyl-4H-naphtho/̲2,3-b̲7pyran-2-carboxylic acid, which comprises hydrolysis of 6,7,8,9-tetrahydro-5-diazo-4-oxo-10-propyl-4H-naphtho/̲2,3-b̲7pyran-2-carboxylic acid or an ester thereof, characterised in that the hydrolysis is carried out in the presence of less than 10% w/w of nitrite anion.

2. A process according to Claim 1, wherein the hydrolysis is carried out in less than 5% w/w of nitrite anion.

3. A process according to Claim 1, wherein the hydrolysis is carried out in the substantial absence of nitrite anion.

4. A process according to any one of the preceding claims, wherein the diazo compound is made by diazotisation of 6,7,8,9-tetrahydro-5-amino-4-oxo-10-propyl-4H-naphtho/̲2,3-b̲7pyran-2-carboxylic acid, or an ester thereof, in the presence of excess nitrite and the excess nitrite is removed or reduced by urea, sulphamic acid or an alcohol.

5. A process according to Claim 4, wherein the excess nitrite is removed or reduced by urea of a C 1 to 6 alkanol.

6. A process according to Claim 5, wherein up to 2 moles of lower alkanol are used per mole of the diazonium compound.

7. A process according to any one of the preceding claims, wherein the hydrolysis is carried out in the presence of urea.

8. A process according to any one of the preceding claims, wherein the hydrolysis is carried out at from $100^{o}$ to $200^{o}$C.

9. A process according to any one of the preceding claims, which

comprises diazotisation of the ethyl ester of 6,7,8,9-tetrahydro-5-amino-4-oxo-10-propyl-4H/̲2,3-b/̲pyran-2-carboxylic acid, adding sufficient ethanol to the reaction mixture to remove excess nitrite and then hydrolysing the resulting mixture in the presence of urea.

10. A process for the production of 6,7,8,9-tetrahydro-5-hydroxy-4-oxo-10-propyl-4H-naphtho/̲2,3-b/̲pyran-2-carboxylic acid, which comprises hydrolysis of 6,7,8,9-tetrahydro-5-diazo-4-oxo-10-propyl-4H-naphtho/̲2,3-b/̲pyran-2-carboxylic acid or an ester thereof, characterised in that the hydrolysis is carried out in such a manner that the product acid crystallises during the hydrolysis.

European Patent Office

# EUROPEAN SEARCH REPORT

0008167

Application number
EP 79301383.0

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.³) 3 |
|---|---|---|---|
| X | DE - A1 - 2 721 021 (FISONS)<br>+ Totality +<br>-- | 1-10 | C 07 D 311/92 |
| X | DE - A1 - 2 722 916 (FISONS)<br>+ Totality +<br>-- | 1-10 | |
| | US - A - 3 886 183 (STRANDTMANN)<br>+ Claims +<br>-- | 1-10 | |
| | US - A - 3 887 585 (KAMINSKY)<br>+ Claims +<br>-- | 1-10 | TECHNICAL FIELDS SEARCHED (Int.Cl.³) 3 |
| | GB - A - 1 416 670 (WARNER)<br>+ Totality +<br>-- | 1-10 | C 07 D 311/92 |
| | DE - A - 2 122 314 (BAYER)<br>+ Claims +<br>-- | 1-10 | |
| | US - A - 3 879 427 (STRANDTMANN)<br>+ Columns 3, 4 +<br>---- | 1-10 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 22-10-1979 | HAMMER |

EPO Form 1503.1 06.78